# FASCICULE DE BREVET EUROPEEN

(11) **EP 1 446 393 B1**
(45) Date de publication et mention de la délivrance du brevet: **08.03.2006**
(21) Numéro de dépôt: 02796839.5
(22) Date de dépôt: 07.11.2002
(51) Int. Cl.: C07D 277/18, C07D 417/12, C07D 417/06, C07D 417/14, A61K 31/427, A61K 31/4439, A61K 31/506, A61K 31/497, A61P 25/16, C07D 295/13, C07D 295/15

(54) **DERIVES DE 2-AMINO-THIAZOLINE ET LEUR UTILISATION COMME INHIBITEURS DE NO-SYNTHASE INDUCTIBLE**
2-AMINO-THIAZOLINDERIVATE UND IHRE VERWENDUNG ALS HEMMSTOFFE DER INDUZIERBAREN NO-SYNTHASE
2-AMINO-THIAZOLINE DERIVATIVES AND THEIR USE AS INHIBITORS OF INDUCIBLE NO-SYNTHASE

(30) Priorité: 09.11.2001 FR 0114510; 30.01.2002 US 352797 P
(43) Date de publication de la demande: 18.08.2004
(73) Titulaire: Aventis Pharma S.A., 92160 Antony (FR)
(72) Inventeur: BIGOT, Antony, F-91300 Massy (FR); CARRY, Jean-Christophe, F-94100 Saint Maur des Fosses (FR); MIGNANI, Serge, F-92290 Chatenay-Malabry (FR)
(74) Mandataire: Rousseau, Pierrick Edouard
(86) Numéro de dépôt international: PCT/FR2002/003810
(87) Numéro de publication internationale: WO 2003/040115

(56) Documents cités:
- WO-A-01/94325
- WO-A-94/12165
- WO-A-95/11231
- WO-A-96/14842

## Description

La présente invention concerne l'utilisation de dérivés de 2-amino-thiazoline de formule : 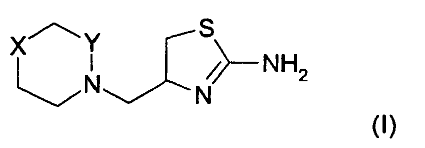
ou leurs sels pharmaceutiquement acceptables comme inhibiteurs de NO-synthase inductible.

L'invention a pour objet l'utilisation des dérivés de 2-amino-thiazoline de formule (I) et leurs sels pharmaceutiquement acceptables pour la préparation de compositions pharmaceutiques destinées à prévenir et traiter les maladies dans lesquelles une production anormale de monoxyde d'azote (NO) par induction de NO-synthase inductible (NOS-2 ou iNOS) est impliquée, les compositions pharmaceutiques contenant les nouveaux dérivés de 2-amino-thiazoline et leurs sels pharmaceutiquement acceptables et les dérivés nouveaux de 2-amino-thiazoline et leurs sels pharmaceutiquement acceptables.

Le monoxyde d'azote (NO) est un radical diffusible impliqué dans de nombreux processus physiologiques et pathologiques. Il est synthétisé par oxydation de la L-arginine, une réaction catalysée par une famille d'enzymes appelée synthase du monoxyde d'azote ou NO-Synthase (NOS), référencée dans le système international de nomenclature des enzymes sous le numéro E.C. 1.14.13.39.

Trois isoformes de NOS, dont deux sont constitutives et une inductible, sont connues :
- une NOS neuronale (NOS-1 ou nNOS) a été isolée et clonée à l'origine à partir de tissu nerveux où c'est une enzyme constitutive. La NOS-1 produit du NO en réponse à divers stimuli physiologiques tels que l'activation de récepteurs membranaires selon un mécanisme dépendant du calcium et de la calmoduline.
- une NOS inductible (NOS-2 ou iNOS) peut être induite en réponse à des stimuli immunologiques tels que par exemple des cytokines ou des antigènes bactériens dans différentes cellules tels que par exemple les macrophages, les cellules endothéliales, les hépatocytes, les cellules gliales, ainsi qu'un grand nombre d'autres types de cellules. L'activité de cette isoforme n'est pas régulée par le calcium. C'est pourquoi une fois induite elle produit de grandes quantités de NO sur des durées prolongées.
- une NOS endothéliale (NOS-3 ou eNOS) est constitutive et calcium/calmoduline dépendante. Elle a été identifiée à l'origine dans les cellules de l'endothélium vasculaire où elle génère du NO en réponse à des stimuli physiologiques tels que l'activation de récepteurs membranaires.

Le NO produit par les isoformes constitutives neuronales et endothéliales (NOS-1 et NOS-3) est généralement impliqué dans des fonctions de signalisation intercellulaire. Par exemple, les cellules endothéliales qui tapissent la paroi interne des vaisseaux sanguins induisent la relaxation des cellules musculaires lisses sous-jacentes *via* la production de NO. Il contribue ainsi à la régulation de la pression artérielle.

Le NO produit en grande quantité par l'isoforme inductible NOS-2 est, entre autre, impliqué dans les phénomènes pathologiques associés aux processus inflammatoires aiguës et chroniques dans une grande variété de tissu et d'organes.

Une production excessive de NO par induction de NOS-2 participe ainsi de pathologies dégénératives du système nerveux comme par exemple la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie. De même, en dehors du système nerveux central, l'induction de NOS-2 est impliquée dans de nombreuses pathologies à composantes inflammatoires comme par exemple le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis. La NOS-2 a également été impliquée dans la croissance de certaines formes de tumeurs comme par exemple des épithéliomes, des adénocarcinomes ou des sarcomes, et dans les infections par des bactéries intracellulaires ou extracellulaires, Gram-plus ou Gram-moins.

Dans toutes les situations où une surproduction de NO est néfaste, il apparaît donc souhaitable de diminuer la production de NO par l'administration de substances capables d'inhiber la NOS-2. Cependant, compte tenu des rôles physiologiques importants joués par l'isoforme constitutive NOS-3 en particulier dans la régulation de la pression artérielle, il est primordial que l'inhibition de l'isoforme NOS-2 affecte le moins possible l'isoforme NOS-3. En effet, il est connu que l'administration d'inhibiteurs non-sélectifs des isoformes de NOS conduit à une vasoconstriction et à un accroissement de la pression artérielle (Moncada, S., Palmer, R.M.J. et Higgs, E.A., Biosynthesis of nitric oxide from L-arginine : a pathway for the regulation of cell function and communication, *Biochem. Pharmacol.,* 1989, 38: 1709-1715). Ces effets sur le système cardiovasculaire sont délétères dans la mesure où ils diminuent l'apport en nutriments aux tissus. Par conséquent, la présente invention concerne des composés présentant une activité inhibitrice vis-à-vis de la NOS-2 significativement plus puissante que son activité inhibitrice vis-à-vis de la NOS-3.

Des inhibiteurs de NOS dérivés de thiazoline sont notamment décrits dans les demandes de brevet WO94/12165, WO95/11231 et WO96/14842.

La présente invention concerne l'utilisation des dérivés de 2-amino-thiazoline de formule (I) dans laquelle :
soit Y est un méthylène (CH₂) et X est choisi parmi les groupement suivants : O, NH, N-Alkyle(C1-C4), N-Bn, N-Ph, N-(2-Py), N-(3-Py), N-(4-Py), N-2-pyrimidyle, N-5-pyrimidyle, S, SO, SO₂, CH₂ ou CHPh ;
soit Y est un carbonyle (C=O) et X est choisi parmi les groupements suivants : NH, N-Ph, N-(2-Py), N-(3-Py), N-(4-Py), N-2-pyrimidyle, N-5-pyrimidyle
   pour la préparation de médicaments utiles pour prévenir ou traiter les maladies dans lesquelles une production anormale de monoxyde d'azote (NO) par induction de NO-synthase inductible (NOS-2 ou iNOS) est impliquée.

Dans les définitions précédentes et celles qui suivent, les radicaux alkyle contiennent 1 à 4 atomes de carbone en chaîne droite ou ramifiée. Les abbréviations Bn, Py, Ph signifient respectivement benzyle, pyridyle, phényle.

Les composés de formule (I) présentent un ou plusieurs carbones asymétriques et peuvent donc se présenter sous forme de racémique, d'énantiomères et de diastéréoisomères; ceux-ci font également partie de l'invention ainsi que leurs mélanges.

Par ailleurs les composés de formule (I) peuvent se présenter sous la forme tautomère (la) :

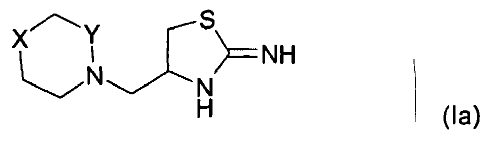

Ces tautomères font également partie de l'invention.

Parmi les composés de formule (I) utiles selon l'invention on peut citer les composés suivants:
4-(morpholin-4-ylméthyl)-4,5-dihydro-1,3-thiazol-2-ylamine
4-(pipérazin-1-ylméthyl)-4,5-dihydro-1,3-thiazol-2-ylamine
4-(4-méthyl-pipérazin-1-ylméthyl)-4,5-dihydro-1,3-thiazol-2-ylamine

leurs racémiques, énantiomères, diastéréoisomères,tautomères ainsi que leurs sels pharmaceutiquement acceptables,

Parmi les composés utiles selon l'invention et particulièrement préférés on peut citer le composé suivant :
4-(4-méthyl-pipérazin-1-ylméthyl)-4,5-dihydro-1,3-thiazol-2-ylamine

son racémique, ses énantiomères, tautomères ainsi que leurs sels pharmaceutiquement acceptables

L'invention concerne également les compositions pharmaceutiques contenant en tant que principe actif un dérivé de formule (I) pour lequel soit Y est un méthylène (CH₂) et X est choisi parmi les groupement suivants : O, NH, N-Alkyle (C1-C4), N-Bn, N-Ph, N-(2-Py), N-(3-Py), N-(4-Py), N-2-pyrimidyle, N-5-pyrimidyle, S, SO, SO₂, CH₂ ou CHPh; soit Y est un carbonyle (C=O) et X est choisi parmi les groupements suivants : NH, N-Ph, N-(2-Py), N-(3-Py), N-(4-Py), N-2-pyrimidyle, N-5-pyrimidyle
ainsi que ses racémiques, énantiomères, diastéréoisomères et leurs mélanges, son tautomère et ses sels pharmaceutiquement acceptables.

Les composés de formule (I) peuvent être préparés par cyclisation d'un dérivé de formule : 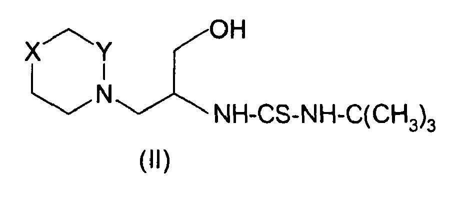
dans laquelle X et Y ont les mêmes significations que dans la formule (I).

Cette cyclisation s'effectue généralement au moyen d'un acide tel que l'acide chlorhydrique, en milieu aqueux, à une température voisine de 100°C. De préférence, on utilise l'acide chlorhydrique 6N.

Les dérivés de formule (II) peuvent être obtenus selon le schéma réactionnel suivant : 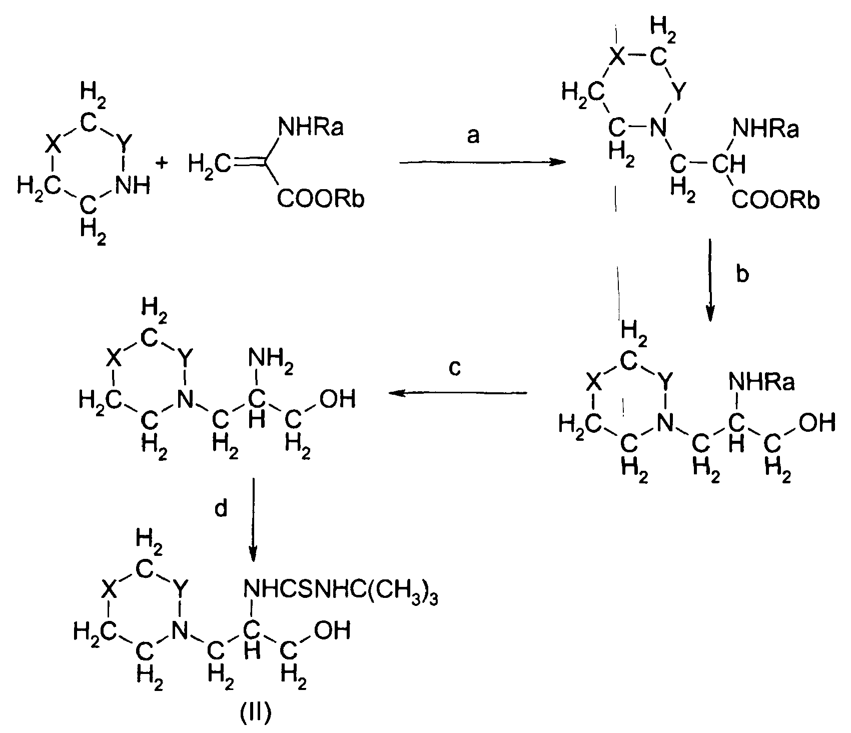
dans ces formules X et Y ont les mêmes significations que dans la formule (I), Ra est un groupe protecteur de la fonction amine tel que ceux décrits par T.W. GREENE, Protective groups in Organic Synthesis, J. Wiley-Interscience Publication (1991), de préférence un radical acétyle ou *tert*-butoxycarbonyle et Rb est un radical alkyle (1-4C) ou alcoxycarbonyle, de préférence, méthyle, éthyle ou isobutyloxycarbonyle.

La réaction a s'effectue généralement en présence d'un acide de Lewis tel que le trichlorure de fer (III), au sein d'un solvant tel que le dichlorométhane ou l'acétonitrile, à une température comprise entre 10°C et la température d'ébullition du milieu réactionnel. Lorsque X représente NH, X peut être mono-protégé au préalable par un groupe protecteur de la fonction amine tel que ceux décrits par T.W. GREENE, Protective groups in Organic Synthesis, J. Wiley-Interscience Publication (1991), de préférence un radical *tert*-butoxycarbonyle

La réaction de réduction b s'effectue de préférence au moyen d'un hydrure tel que le borohydrure de sodium ou l'aluminohydrure de lithium, au sein d'un alcool aliphatique (1-4C) ou du tétrahydrofuranne, à une température comprise entre 0°C et 30°C.

La réaction de déprotection c s'effectue avec un agent de déprotection pour les composés pour lesquels Ra est un groupe protecteur de la fonction amine par toute méthode de déprotection connue de l'homme de l'art et notamment celles décrites par T.W. GREENE, Protective groups in Organic Synthesis, J. Wiley-Interscience Publication (1991). De préférence lorsque le groupe protecteur est un radical acétyle cette réaction s'effectue au moyen d'acide chlorhydrique aqueux, à une température voisine de 100°C. Lorsque le groupe protecteur est un radical *tert*-butoxycarbonyle cette réaction s'effectue au moyen d'acide chlorhydrique au sein du dioxanne, à une température voisine de 20°C.

La réaction d s'effectue par action du *tert-*butyl isothiocyanate, au sein d'un solvant inerte tel qu'un alcool aliphatique (1-4C) (méthanol, éthanol de préférence), éventuellement en présence d'une amine tertiaire telle que la triéthylamine, à une température comprise entre 20°C et la température d'ébullition du milieu réactionnel.

Les composés de formule (I) pour lesquels X représente soit SO, soit SO₂ peuvent être obtenus par oxydation directe du composé de formule (I) pour lequel X représente S. Cette oxydation s'effectue selon les méthodes connues d'oxydation des dérivés soufrés comme celles décrites par M. HUDLICKY, Oxidation in Organic Chemistry, ACS Monograph, 186, 252-263 (1990). Par exemple, on opère par action d'un peracide organique ou un sel d'un peracide organique (acide percarboxylique ou persulfonique, notamment l'acide perbenzoïque, l'acide 3-chloro-perbenzoïque, l'acide 4-nitroperbenzoïque, l'acide peracétique, l'acide pertrifluoroacétique, l'acide performique, l'acide monoperphthalique) ou un peracide minéral ou un sel d'un peracide minéral (par exemple l'acide periodique ou persulfurique), au sein d'un solvant inerte tel qu'un solvant chloré (chloroforme, dichlorométhane par exemple), à une température comprise entre 0 et 20°C. On peut utiliser également le peroxyde d'hydrogène ou un periodate (periodate de sodium par exemple), au sein d'un solvant inerte tel qu'un alcool aliphatique inférieur, l'eau ou un mélange de ces solvants, à une température comprise entre 0 et 20°C. Ces produits peuvent être aussi préparés à partir des composés de formule (II) correspondants, eux-mêmes obtenus selon le schéma réactionnel suivant :

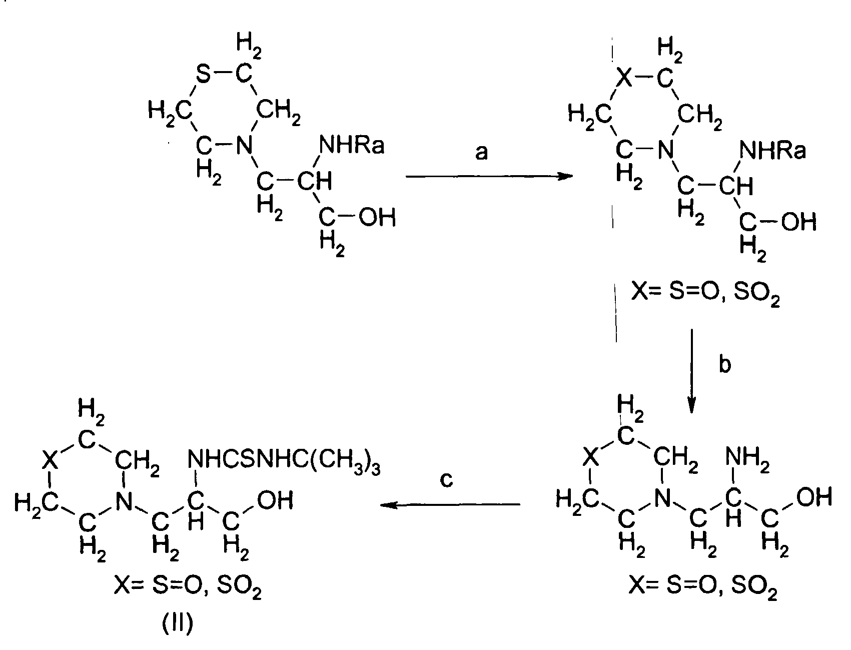

La réaction d'oxydation a s'effectue selon les méthodes connues d'oxydation des dérivés soufrés comme celles décrites ci-dessus.

La réaction de déprotection b pour les composés pour lesquels Ra est un groupe protecteur de la fonction amine s'effectue par toute méthode de déprotection connue de l'homme de l'art et notamment celles décrites par T.W. GREENE, Protective groups in Organic Synthesis, J. Wiley-Interscience Publication (1991). De préférence lorsque le groupe protecteur est un radical acétyle cette réaction s'effectue au moyen d'acide chlorhydrique aqueux, à une température voisine de 100°C. Lorsque le groupe protecteur est un radical *tert*-butoxycarbonyle cette réaction s'effectue au moyen d'acide chlorhydrique au sein du dioxanne, à une température voisine de 20°C.

La réaction c s'effectue par action du tert-butyl isothiocyanate, au sein d'un solvant inerte tel qu'un alcool aliphatique (1-4C) (méthanol, éthanol de préférence), éventuellement en présence d'une amine tertiaire telle que la triéthylamine, à une température comprise entre 20°C et la température d'ébullition du milieu réactionnel.

Les composés de formule (I) sont isolés et peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) sont des inhibiteurs de NO-synthase inductible ou NO-synthase de type 2 (NOS-2) et sont ainsi utiles pour la prévention et le traitement des désordres liés à une production excessive de NO telles que la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uveite, le syndrome de Guillain-Barré, la glomerulo-néphrite, le lupus erythémateux, le psoriasis, la croissance de certaines formes de tumeurs comme par exemple les épithéliomes, les adénocarcinomes ou les sarcomes, et les infections par des bactéries intracellulaires ou extracellulaires, gram-plus ou gram-moins.

Leurs activités en tant qu'inhibiteurs de NOS-2 et NOS-3 ont été déterminées par la mesure de la conversion de [³H]-L-arginine en [³H]-L-citrulline par, respectivement, une fraction enzymatique NOS-2 extraite de poumons de rats ou de souris préalablement traités par des lipopolysaccharides (10 mg/kg i.p. 6 heures avant le recueil de tissu) et par une préparation commerciale de NOS-3 recombinante de boeuf. Les composés ont été incubés pendant 20 à 30 minutes à 37°C en présence de 5 µM (pour activité NOS-2) ou 10 µM (pour activité NOS-3) de [³H]-L-arginine, 1 mM de NADPH, 15 µM de tétrabiopterine, 1µM de FAD, 0.1 mM de DTT dans un tampon HEPES (50 mM, pH 6,7) contenant 10 µg/ml de calmoduline et 1.25 mM de CaCl₂ lorsque l'activité NOS-3 a été mesurée. L'incubation a été arrêtée par addition de tampon HEPES froid (100 mM, pH 5,5) contenant 10 mM d'EGTA et 500 mg d'une résine cationique échangeuse d'ion (AG50W-X8, contre-ion : Na⁺) pour séparer la [³H]-L-arginine de la [³H]-L-citruiline. Après 5 min de décantation, la radioactivité restant dans la phase liquide a été mesurée dans un compteur à scintillation en présence d'un liquide scintillant approprié. Le rendement de la récupération de la L-[³H]citrulline formée a pu être estimée en utilisant de la L-[ureido-¹⁴C]-Citrulline comme standard externe.
L'activité NOS-2 ou NOS-3 a été exprimée en picomole(s) de [³H]-L-citrulline formée par minute et par milligramme de protéine contenu dans le milieu réactionel.

Dans ce test sur l'enzyme NOS-2, la CI₅₀ des composés de formule (I) est inférieure ou égale à 10 µM.

La sélectivité est mesurée par le rapport CI₅₀ NOS-3 / CI₅₀ NOS-2. Cette sélectivité est supérieure à 45.

Les composés de formule (1) présentent une toxicité faible. Leur DL₅₀ est supérieure à 40 mg/kg par voie sous cutanée chez la souris.

Les exemples suivants illustrent l'invention.

### Exemple 1 :

### 4-(4-Méthyl pipérazin-1-ylméthyl)-4,5-dihydro-thiazol-2-ylamine trichlorhydrate

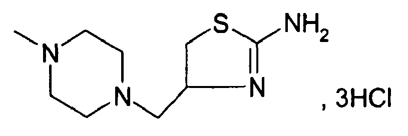

Une suspension de 0,42 g de *N-*(*tert-butyl*)-N'-[2-hydroxy-1-(4-méthyl-pipérazin-1-ylméthyl)éthyl]-thiourée dans 3,9 cm³ d'une solution aqueuse d'acide chlorhydrique 6N est chauffée à une température voisine de 100°C pendant 5 heures. Après refroidissement, le milieu réactionnel est ensuite concentré à sec sous pression réduite (2 kPa) à une température voisine de 55°C. La meringue obtenue est séchée dans un dessiccateur sous vide (2 kPa) pendant 4 heures. On obtient ainsi 0,47 g de 4-(4-méthyl-pipérazin-1-ylméthyl)-4,5-dihydro-thiazol-2-ylamine, trichlorhydrate sous forme d'une meringue de couleur blanc cassé très hygroscopique. Spectre de R.M.N. ¹H (300 MHz, (CD₃)₂SO d6 avec ajout de quelques gouttes de CD₃COOD d4, δ en ppm) : de 2,55 à 2,90 (mf: 4H) ; 2,80 (s : 3H) ; de 2,95 à 3,30 (mf: 4H) ; de 3,30 à 3,60 (mf : 2H) ; 3,40 (dd, J = 11,5 et 5,5 Hz : 1H); 3,69 (dd, J = 11,5 et 7,5 Hz : 1H); 4,51 (mt : 1H).

### N-(tert-Bulyl)-N'-[2-hydroxy-1-(4-méthyl-pipérazin-1-ylméthyl)éthyl]-thiourée

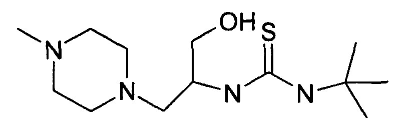

A une solution de 1 g de 2-amino-3-(4-méthyl-pipérazinl-yl)-1-propanol chlorhydrate dans 20 cm³ d'éthanol anhydre et 1,43 cm³ de triéthylamine, on ajoute 0,78 cm³ de *tert*-butylisothiocyanate. Le mélange réactionnel est agité sous atmosphère inerte à une température voisine de 20°C pendant 42 heures puis est chauffé à une température voisine de 50°C pendant 1 heure 30 min. Après refroidissement à une température voisine de 20°C, le milieu réactionnel est évaporé sous pression réduite (2 kPa) à une température voisine de 30°C. Le résidu d'évaporation est repris dans 10 cm³ d'eau et 40 cm³ de dichlorométhane. La phase aqueuse est extraite par 2 fois 30 cm³ de dichlorométhane. Les phases organiques sont réunies, lavées par 15 cm³ d'eau, séchées sur sulfate de magnésium, filtrées puis évaporées sous pression réduite (2 kPa) à une température voisine de 20°C. On obtient ainsi 0,42 g de *N-(tert*-butyl)-N'-[2-hydroxy-1-(4-méthyl-pipérazin-1-ylméthyl)éthyl]thiourée, sous forme d'une meringue blanche. Spectre infra-rouge entre lamelles de KBr 3279 ; 3075 ; 2939 ; 2806 ; 1533 ; 1459 ; 1359 ; 1295 ; 1204 ; 1010 et 821 cm⁻¹.

### 2-Amino-3-(4-méthyl-pipérazin-1-yl)-1-propanol chlorhydrate

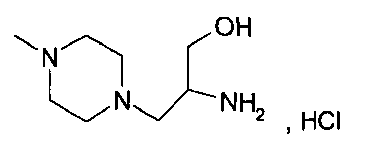

Une suspension de 0,89 g de *N*-[2-hydroxy-1-(4-méthyl-pipérazin-1-ylméthyl)éthyl] acétamide dans 10,3 cm³ d'une solution aqueuse d'acide chlorhydrique 6N est chauffée à une température voisine de 100°C pendant 3 heures. Après refroidissement à une température voisine de 60°C, le milieu réactionnel est filtré et le filtrat est évaporé sous pression réduite (2 kPa) à une température voisine de 60°C. On obtient ainsi 1 g de 2-amino-3-(4-méthyl-pipérazinyl)-1-propanol, chlorhydrate sous forme d'une meringue collante de couleur beige.
Spectre IR (KBr) 3337 ; 2955 ; 2637 ; 2522 ; 1617 ; 1457 ; 1062 ; 1009 et 962 cm⁻¹.

### N-[2-Hydroxy-1-(4-méthyl-pipérazin-1-ylméthyl)éthyl] acétamide

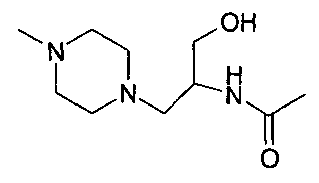

Une solution sous atmosphère inerte de 3,27 g de 2-(acétylamino)-3-(4-méthyl-pipérazin-1-yl)propanoate de méthyle dans 100 cm³ de méthanol anhydre est refroidie à une température voisine de 10°C, puis on ajoute par spatulées 0,76 g de borohydrure de sodium. Le mélange réactionnel est agité pendant 5 heures à une température voisine de 20°C, puis on ajoute de nouveau 0,26 g de borohydrure de sodium et l'agitation est poursuivie pendant 38 heures. On coule ensuite 5 cm³ d'eau sur la masse réactionnelle qui est ensuite concentrée sous pression réduite (2 kPa) à une température voisine de 30°C. La pâte résiduelle obtenue est triturée avec du dichlorométhane et l'insoluble est filtré. Le filtrat est concentré sous pression réduite (2 kPa) à une température voisine de 20°C. Le résidu est purifiée par chromatographie sous pression d'argon (60 kPa), sur une colonne de gel de silice (granulométrie 40-63 µm; diamètre 5 cm ; hauteur 19 cm), en éluant par des mélanges successifs de dichlorométhane-méthanol-ammoniaque à 20% (98/2/0, 95/5/0,1, 90/10/0,2, 80/20/0,25, 50/50/0,25 en volumes). Les fractions contenant le produit attendu sont réunies et concentrées sous pression réduite (2 kPa) à une température voisine de 40°C. On obtient ainsi 0,92 g de *N*-[2-hydroxy-1-(4-méthyl-pipérazin-1-ylméthyl)éthyl] acétamide, sous forme d'un liquide jaune. Spectre IR CH₂Cl₂ 3621; 3429; 3352; 2944; 2803; 1657; 1513; 1460; 1284; 1050; 1011 et 816 cm⁻¹.

### 2-(Acétylamino)-3-(4-méthyl-pipérazin-1-yl)propanoate de méthyle

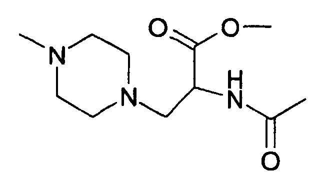

A une solution de 8,57 g de 2-acétamidoacrylate de méthyle dans 500 cm³ de dichlorométhane agitée sous atmosphère inerte, on ajoute 6,65 cm³ de *N-*méthylpipérazine puis 0,97 g de trichlorure de fer et l'ensemble est agité au voisinage de 20°C pendant 66 heures. On coule alors 300 cm³ d'une solution aqueuse de sulfate de sodium sur le mélange réactionnel qui est agité vivement puis filtré sur de la Célite. Après décantation, la phase organique est séchée sur du sulfate de sodium, filtrée puis concentrée à sec sous pression réduite (2 kPa) à une température voisine de 40°C pour donner un liquide de couleur orangée. La phase aqueuse est extraite par 3 fois 150 cm³ de dichlorométhane et tous les extraits organiques sont réunis, séchés sur du sulfate de sodium, puis concentrés sous pression réduite (2 kPa) à une température voisine de 20°C pour donner une huile jaune. Les deux intermédiaires issus des deux phases organiques sont réunis et purifiés par chromatographie sous pression d'argon (50 kPa), sur une colonne de gel de silice (granulométrie 40-63 µm ; diamètre 5 cm ; hauteur 25 cm), en éluant par des mélanges successifs de dichlorométhane-méthanol-ammoniaque à 20% (99/1/0, 97/3/0, 90/10/0,25, 80/20/0,25 en volumes). Les fractions contenant le produit attendu sont réunies et concentrées sous pression réduite (2 kPa) à une température voisine de 30°C. On obtient ainsi 3,3 g de 2-(acétylamino)-3-(4-méthyl-pipérazinyl)propanoate de méthyle sous forme d'un liquide jaune. Spectre IR CC1₄ 3437; 3318; 2941; 2798; 1749; 1685; 1499; 1458; 1374; 1286; 1204; 1168 et 1014 cm⁻¹.

Les compositions pharmaceutiques selon l'invention sont constitués par un composé de formule (I) ou un isomère ou un tautomère ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops ets élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention de la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis, la croissance de certaines formes de tumeurs comme par exemple les épithéliomes, les adénocarcinomes ou les sarcomes, et les infections par des bactéries intracellulaires ou extracellulaires, Gram-plus ou Gram-moins.

Les doses dépendent de l'effet recherché, de la durée du traitement et de la voie d'administration utilisée; elles sont généralement comprises entre 1 mg et 100 mg par jour par voie orale pour un adulte avec des doses unitaires allant de 0,5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composés de formule (I) 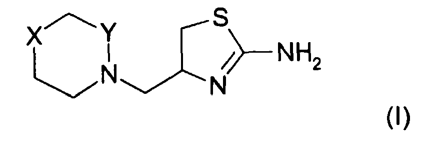
dans laquelle
soit Y est un méthylène (CH₂) et X est choisi parmi les groupement suivants : O, NH, N-Alkyle(C1-C4), N-Bn, N-Ph, N(2-Py), N-(3-Py), N-(4-Py), N-2-pyrimidyle, N-5-pyrimidyle, S, SO, SO₂, CH₂ ou CHPh ;
soit Y est un carbonyle (C=O) et X est choisi parmi les groupements suivants : NH, N-Ph, N-(2-Py), N-(3-Py), N-(4-Py), N-2-pyrimidyle ou N-5-pyrimidyle
étant entendu que les radicaux alkyle(C1-C4) renferment 1 à 4 carbones en chaîne droite ou ramifiée. Les abbréviations Bn, Py, Ph signifient respectivement benzyle, pyridyle, phényle
leurs racémiques, énantiomères, diastéréoisomères et leurs mélanges, leurs tautomères et leurs sels pharmaceutiquement acceptables.

2. Composé selon la revendication 1 **caractérisé par le fait qu'**il est choisi parmi :
4-(morpholin-4-ylméthyl)-4,5-dihydro-1,3-thiazol-2-ylamine
4-(pipérazin-1-ylméthyl)-4,5-dihydro-1,3-thiazol-2-ylamine
4-(4-méthyl-pipérazin-1-ylméthyl)-4,5-dihydro-1,3-thiazol-2-ylamine
leur racémiques, énantiomères, diastéréoisomères et leurs mélanges, leurs tautomères et leurs sels pharmaceutiquement acceptables.

3. Composé selon l'une quelconque des revendications 1 à 2 **caractérisé par le fait qu'**il est choisi parmi :
4-(4-méthyl-pipérazin-1-ylméthyl)-4,5-dihydro-1,3-thiazol-2-ylamine
son racémique, ses énantiomères, tautomères ainsi que leurs sels pharmaceutiquement acceptables.

4. Composé selon l'une quelconque des revendications 1 à 3 utilisé pour préparer un médicament.

5. Composition pharmaceutique **caractérisée par le fait qu'**elle comprend dans un milieu pharmaceutiquement acceptable, un composé défini selon l'une quelconque des revendications 1 à 3.

6. Médicament selon la revendication 4 **caractérisé par le fait qu'**il contient au moins un composé défini selon l'une quelconque des revendications 1 à 3 pour son application thérapeutique dans le traitement des maladies dans lesquelles une production anormale de monoxyde d'azote (NO) par induction de NO-synthase inductible (NOS-2) est impliquée.

7. Médicament selon la revendication 4 **caractérisé par le fait qu'**il contient au moins un composé défini selon l'une quelconque des revendications 1 à 3 pour son application thérapeutique dans le traitement de la maladie de Parkinson.

8. Procédé de préparation des composés de formule (I) tels que définis dans la revendication 1 **caractérisé en ce que** l'on cyclise un dérivé de formule : 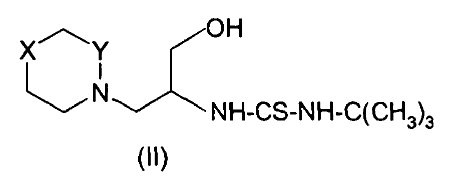
dans laquelle X et Y ont les mêmes significations que dans la revendication 1.

9. Procédé de préparation selon la revendication 8 **caractérisé en ce que** la cyclisation se fait en milieu acide à une température voisine de 100°C.

10. Procédé de préparation selon la revendication 9 **caractérisé en ce que** le milieu acide est préférentiellement de l'acide chlorhydrique 6N

11. Procédé de préparation des composés de formule (II) telle que définie à la revendication 8 et dans laquelle X et Y ont la même signification qu'à la revendication 1 **caractérisé en ce qu'**on soumet un composé de formule (IIa) 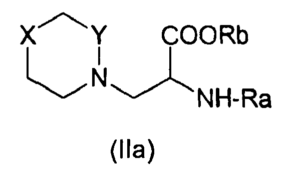
Ra étant un groupe protecteur de la formation amine et Rb un radical alkyl(1-4C) ou alcoxycarbonyle à l'action d'un agent de réduction afin d'obtenir le composé de formule (IIb) 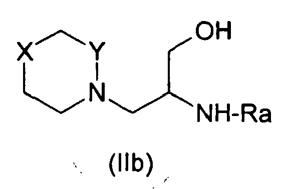
que l'on soumet à l'action d'un agent de déprotection afin d'obtenir un composé de formule (IIc) 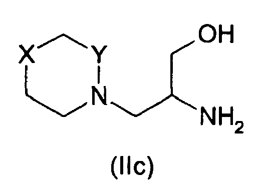
que l'on soumet à l'action du *tert*-butylisothiocyanate afin d'obtenir un composé de formule (II)

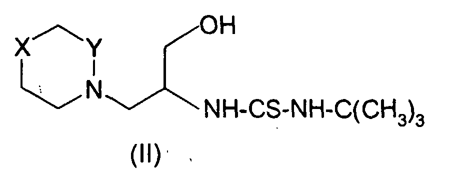

12. A titre de produits intermédiaires, les composés de formules II, IIa, IIb, IIc telle que définies dans la revendication 11 à l'exception des produits suivants :
N-[2-hydroxy-1-(4-phényl-pipérazin-1-ylmethyl)ethyl] thiobenzamide
methyl 2-(N-CBZ-amino)-3-morpholino-propionate
boc-DL-β-4-morpholinyl)alanine methyl ester
methyl 2-acetamido-3-piperidinopropanoate
methyl 2-acetamido-3-morpholinopropanoate
éthyl 2-(N-benzyl-amino)-3-piperidinopropanoate
éthyl 2-(N-benzyl-amino)-3-morpholinopropanoate

13. A titre de produits intermédiaires *N-(tert*-Butyl)-N'-[2-hydroxy-1-(4-méthyl-pipérazin-1-ylméthyl)éthyl]-thiourée ; *N*-[2-Hydroxy-1-(4-méthyl-pipérazin-1-ylméthyl)éthyl] acétamide ; 2-(Acétylamino)-3-(4-méthyl-pipérazin-1-yl)propanoate de méthyle

## Claims

1. Compounds of formula (I) 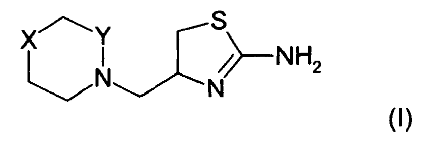
in which
either Y is a methylene (CH₂) and X is selected from the following groups: O, NH, N-(C1-C4)alkyl, N-Bn, N-Ph, N-(2-Py), N-(3-Py), N-(4-Py), N-2-pyrimidyl, N-5-pyrimidyl, S, SO, SO₂, CH₂ or CHPh;
or Y is a carbonyl (C=O) and X is selected from the following groups: NH, N-Ph, N-(2-Py), N-(3-Py), N-(4-Py), N-2-pyrimidyl or N-5-pyrimidyl
with the proviso that the (C1-C4)alkyl radicals contain 1 to 4 carbons in a straight or branched chain. The abbreviations Bn, Py and Ph signify benzyl, pyridyl and phenyl respectively.
their racemic mixtures, enantiomers, diastereoisomers and mixtures thereof, their tautomers and their pharmaceutically acceptable salts.

2. Compound according to Claim 1, **characterized in that** it is selected from:
4-(morpholin-4-ylmethyl)-4,5-dihydro-1,3-thiazol-2-ylamine
4-(piperazin-1-ylmethyl)-4,5-dihydro-1,3-thiazol-2-ylamine
4-(4-methyl-piperazin-1-ylmethyl)-4,5-dihydro-1,3-thiazol-2-ylamine
their racemic mixtures, enantiomers, diastereoisomers and mixtures thereof, their tautomers and their pharmaceutically acceptable salts.

3. Compound according to any one of Claims 1 to 2, **characterized in that** it is selected from:
4-(4-methyl-piperazin-1-ylmethyl)-4,5-dihydro-1,3-thiazol-2-ylamine
its racemic mixtures, its enantiomers and tautomers and their pharmaceutically acceptable salts.

4. Compound according to any one of Claims 1 to 3 used to prepare a medicament.

5. Pharmaceutical composition **characterized in that** it comprises in a pharmaceutically acceptable medium a compound defined according to any one of Claims 1 to 3.

6. Medicament according to Claim 4, **characterized in that** it contains at least one compound defined according to any one of Claims 1 to 3 for its therapeutic application in the treatment of diseases involving abnormal production of nitrogen monoxide (NO) by induction of inducible NO synthase (NOS-2).

7. Medicament according to Claim 4, **characterized in that** it contains at least one compound defined according to any one of Claims 1 to 3 for its therapeutic application in the treatment of Parkinson's disease.

8. Process for preparing compounds of formula (I) as defined in Claim 1, **characterized in that** a derivative of formula: 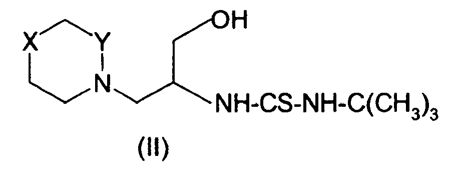
in which X and Y have the same meanings as in Claim 1 is cyclized.

9. Preparation process according to Claim 8, **characterized in that** the cyclization takes place in an acidic medium at a temperature in the region of 100°C.

10. Preparation process according to Claim 9, **characterized in that** the acidic medium is preferably 6N hydrochloric acid.

11. Process for preparing compounds of formula (II) as defined in Claim 8 and in which X and Y have the same meaning as in Claim 1, **characterized in that** a compound of formula (IIa) 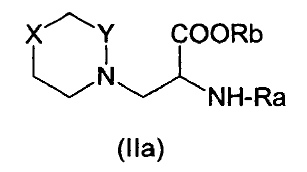
Ra being a protective group for the amine formation and Rb a (1-4C) alkyl radical or alkoxycarbonyl radical, is subjected to the action of a reducing agent so as to give he compound of formula (IIb) 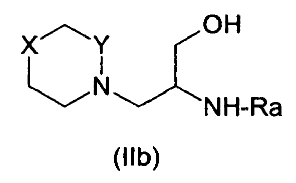
which is subjected to the action of a deprotection agent so as to give a compound of formula (IIc) 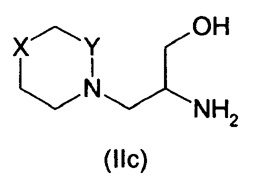
which is subjected to the action of *tert*-butyl isothiocyanate so as to give a compound of formula (II)

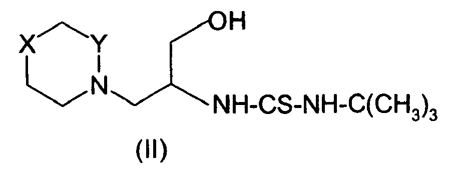

12. As intermediates, the compounds of formulae II, IIa, IIb and IIc as defined in Claim 11 with the exception of the following products:
N-[2-hydroxy-1-(4-phenylpiperazin-1-ylmethyl)ethyl]thiobenzamide
methyl 2-(N-CBZ-amino)-3-morpholino-propionate
boc-DL-β-4-morpholinyl)alanine methyl ester
methyl 2-acetamido-3-piperidinopropanoate
methyl 2-acetamido-3-morpholinopropanoate
ethyl 2-(N-benzyl-amino)-3-piperidinopropanoate
ethyl 2-(N-benzyl-amino)-3-morpholinopropanoate.

13. As intermediates, *N*-(*tert-*butyl)-N'-[2-hydroxy-1-(4-methylpiperazin-1-ylmethyl)ethyl]thiourea; *N*-[2-hydroxy-1-(4-methylpiperazin-1-ylmethyl)ethyl]-acetamide; methyl 2-(acetylamino)-3-(4-methylpiperazin-1-yl)propanoate.

## Patentansprüche

1. Verbindungen der Formel (I) 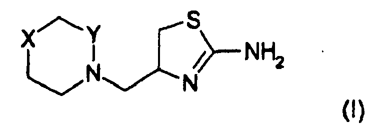
in der
entweder Y ein Methylen (CH₂) ist und X ausgewählt wird unter den folgenden Gruppen: O, NH, N-Alkyl(C1-C4), N-Bn, N-Ph, N-(2-Py), N-(3-Py), N-(4-Py), N-2-pyrimidyl, N-5-pyrimidyl, S, SO, SO₂, CH₂ oder CHPh;
oder Y ein Carbonyl (C=O) ist und X ausgewählt wird unter den folgenden Gruppen: NH, N-Ph, N-(2-Py), N-(3-Py), N-(4-Py), N-2-pyrimidyl oder N-5-pyrimidyl,
mit der Maßgabe, daß die Reste Alkyl(C1-C4) 1 bis 4 Kohlenstoffatome in gerader oder verzweigter Kette umfassen und die Abkürzungen Bn, Py, Ph jeweils Benzyl, Pyridyl, Phenyl kennzeichnen,
sowie ihre Racemate, Enantiomeren, Diastereoisomeren und ihre Mischungen, ihre Tautomeren und ihre pharmazeutisch akzeptablen Salze.

2. Verbindung nach Anspruch 1, **dadurch gekennzeichnet, daß** sie ausgewählt wird unter:
4-(Morpholin-4-ylmethyl)-4,5-dihydro-1,3-thiazol-2-ylamin
4-(Piperazin-1-ylmethyl)-4,5-dihydro-1,3-thiazol-2-ylamin
4-(4-Methyl-piperazin-1-ylmethyl)-4,5-dihydro-1,3-thiazol-2-ylamin
sowie ihre Racemate, Enantiomeren, Diastereoisomeren und ihre Mischungen, ihre Tautomeren und ihre pharmazeutisch akzeptablen Salze.

3. Verbindung nach irgendeinem der Ansprüche 1 bis 2, **dadurch gekennzeichnet, daß** sie ausgewählt wird unter:
4-(4-Methyl-piperazin-1-ylmethyl)-4,5-dihydro-1,3-thiazol-2-ylamin,
ihren Racematen, ihren Enantiomeren, Tautomeren sowie ihren pharmazeutisch akzeptablen Salzen.

4. Verbindung nach irgendeinem der Ansprüche 1 bis 3, verwendet zur Herstellung eines Arzneimittels.

5. Pharmazeutische Zusammensetzung, **dadurch gekennzeichnet, daß** sie in einem pharmazeutisch akzeptablen Medium eine Verbindung, wie in irgendeinem der Ansprüche 1 bis 3 definiert, umfaßt.

6. Arzneimittel nach Anspruch 4, **dadurch gekennzeichnet, daß** es mindestens eine Verbindung, wie in irgendeinem der Ansprüche I bis 3 definiert, enthält für seine therapeutische Anwendung bei der Behandlung von Erkrankungen, bei denen eine anormale Produktion von Stickstoffmonoxid (NO) durch Induktion von induktibler NO-Synthase (NOS-2) einbezogen ist.

7. Arzneimittel nach Anspruch 4, **dadurch gekennzeichnet, daß** es mindestens eine Verbindung, wie in irgendeinem der Ansprüche 1 bis 3 definiert, enthält für seine therapeutische Anwendung bei der Behandlung der Parkinson-Erkrankung.

8. Verfahren zur Herstellung der Verbindungen der Formel (I) wie in Anspruch 1 definiert, **dadurch gekennzeichnet, daß** man ein Derivat der Formel (II) 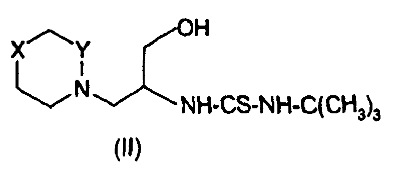
cyclisiert, in der X und Y die gleichen Bedeutungen besitzen wie in Anspruch 1.

9. Verfahren zur Herstellung nach Anspruch 8, **dadurch gekennzeichnet, daß** die Cyclisierung im sauren Medium bei einer Temperatur von etwa 100 °C erfolgt.

10. Verfahren nach Anspruch 9, **dadurch gekennzeichnet, daß** das saure Medium vorzugsweise 6 N Chlorwasserstoffsäure ist.

11. Verfahren zur Herstellung der Verbindungen der Formel (II) wie in Anspruch 8 definiert und worin X und Y die gleiche Bedeutung besitzen wie in Anspruch 1, **dadurch gekennzeichnet, daß** man eine Verbindungen der Formel (IIa) 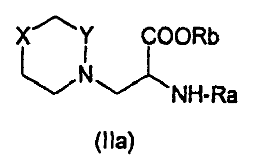
worin Ra eine Schutzgruppe für die Aminformation ist und Rb einen Rest Alkyl(1-4C) oder Alkoxycarbonyl darstellt, der Einwirkung eines Reduktionsmittels unterzieht, um die Verbindung der Formel (IIb) 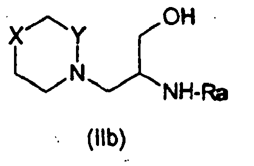
zu erhalten, die man der Einwirkung eines Mittels zur Abspaltung der Schutzgruppe unterzieht, um eine Verbindung der Formel (IIc) 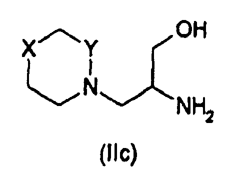
zu erhalten, die man der Einwirkung von tert.-Butylisothiocyanat unterzieht, um eine Verbindung der Formel (II) 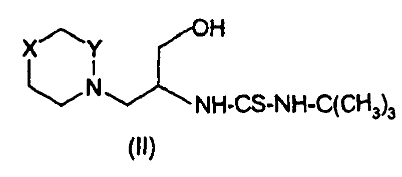
zu erhalten.

12. Als Zwischenprodukte die Verbindungen der Formeln (II), (IIa), (IIb), (IIc) wie in Anspruch 11 definiert, mit Ausnahme der folgenden Produkte:
N-[2-Hydroxy-1-(4-phenyl-piperazin-1-ylmethyl)-ethyl]-thio-benzamid
Methyl-2-(N-CBZ-amino)-3-morpholino-propionat
Boc-DL-β-4-morpholinyl-alanin-methylester
Methyl-2-acetamido-3-piperidino-propanoat
Methyl-2-acetamido-3-morpholino-propanoat
Ethyl-2-(N-benzyl-amino)-3-piperidino-propanoat
Ethyl-2-(N-benzyl-amino)-3-morpholino-propanoat

13. Als Zwischenprodukte N-(tert.-Butyl)-N'-[2-hydroxy-1-(4-methyl-piperazin-1-ylmethyl)-ethyl]-thioharnstoff; N-[2-Hydroxy-1-(4-methyl-piperazin-1-ylmethyl)-ethyl]-acetamid; 2-(Acetylamino)-3-(4-methyl-piperazin-1-yl)-propansäure-methylester.
